(19) Europäisches Patentamt
European Patent Office
Office européen des brevets

(11) **EP 2 252 231 B1**

(12) **EUROPEAN PATENT SPECIFICATION**

(45) Date of publication and mention
of the grant of the patent:
**16.10.2019 Bulletin 2019/42**

(51) Int Cl.:
***A61B 34/30*** (2016.01)   ***B25J 18/00*** (2006.01)

(21) Application number: **09720393.9**

(22) Date of filing: **11.03.2009**

(86) International application number:
**PCT/US2009/036802**

(87) International publication number:
**WO 2009/114613 (17.09.2009 Gazette 2009/38)**

(54) **SYSTEM AND METHOD FOR ROBOTIC SURGERY SIMULATION**

SYSTEM UND VERFAHREN FÜR EINE ROBOTER-CHIRURGIESIMULATION

PROCÉDÉ ET SYSTÈME POUR SIMULATION DE CHIRURGIE ROBOTISÉE

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **11.03.2008 US 35594 P**

(43) Date of publication of application:
**24.11.2010 Bulletin 2010/47**

(73) Proprietors:
- **Health Research, INC.
  Buffalo, NY 14263 (US)**
- **The Research Foundation Of State University
  Of New York
  Amherst, NY 14228-2567 (US)**

(72) Inventors:
- **GURU, Khurshid
  East Amherst
  NY 14051 (US)**
- **KESAVADAS, Thenkurussi
  Clarence Center
  NY 14032 (US)**
- **SRIMATHVEERAVALLI, Govindarajan
  Buffalo
  NY 14222 (US)**

(74) Representative: **Appleyard Lees IP LLP
15 Clare Road
Halifax HX1 2HY (GB)**

(56) References cited:
**US-A1- 2003 109 780   US-A1- 2004 009 459
US-A1- 2004 066 369   US-A1- 2006 232 664
US-A1- 2007 238 081   US-A1- 2008 004 632**

- **L. W. SUN ET AL: "Advanced da Vinci surgical
  system simulator for surgeon training and
  operation planning", INTERNATIONAL
  JOURNAL OF MEDICAL ROBOTICS AND
  COMPUTER ASSISTEDSURGERY, vol. 3, no. 3,
  19 June 2007 (2007-06-19), pages 245-251,
  XP055097699, GB ISSN: 1478-5951, DOI:
  10.1002/rcs.139**
- **TAHMASEBI A M ET AL: "Dynamic parameter
  identification and analysis of a PHANToM haptic
  device", CONTROL APPLICATIONS, 2005. CCA
  2005. PROCEEDINGS OF 2005 IEEE CONFERE
  NCE ON TORONTO, CANADA AUG. 29-31, 2005,
  PISCATAWAY, NJ, USA,IEEE, 29 August 2005
  (2005-08-29), pages 1251-1256, XP010835263,
  DOI: 10.1109/CCA.2005.1507303 ISBN:
  978-0-7803-9354-7**
- **'Proceedings of the 2007 IEEE International
  Conference on Mechatronics and Automation.
  5-8 August 2007', 2007 article LOI-WAH SUN ET
  AL.: 'Design and Development of a Da Vinci
  Surgical System Simulator.', pages 1050 - 1055,
  XP031134889**

EP 2 252 231 B1

**Description**

**Field of the Invention**

The present invention relates generally to robotic surgery simulators and in particular, to a system and method for simulating the kinematics of a surgical robot.

**Background of the Invention**

Robotic surgery is becoming increasingly popular due to its many benefits over traditional open surgeries, including quicker recovery time, less pain, and less scarring. A robotic surgery system, such as the da Vinci® Surgical System ("dVSS") from Intuitive Surgical, Inc., typically consists of two main components; master console and a slave robot. A surgeon provides input through a manipulator of the master console which, in turn, controls a slave robot to perform the necessary motions at the patient.

Many surgeons are reluctant to switch to surgical robots, however, because of the differences in the skill set of the surgeon. For example, in traditional open surgeries and laparoscopic surgical procedures, the forces encountered by the tools used in the surgery are felt by the surgeon. In contrast, most robotic master consoles do not provide any force feedback to the surgeon. Generations of surgeons have been trained to perform surgical procedures using tactile sensation as a key sensory input, and performing a procedure without this sense is seen as a paradigm shift requiring extensive re-training before a surgeon may be allowed to perform procedures using robotic systems.

The acquisition costs for surgical robots is high-as high as several million dollars. Because of the expense of the equipment, it is most cost effective to devote as much of the surgical robot's time to performance of actual procedures. Therefore, the availability of such expensive equipment for training is usually low.

Accordingly, there exists a need for a less expensive system for training surgeons in robotic procedures. Such a system should: (1) provide articulated input devices such that the kinesthetics of working with the master console is preserved; (2) provide accurate training simulations to minimize training time; (3) be compact and easy to assemble and disassemble so that the system may be transported to any location convenient for training; and (4) be inexpensive to manufacture.

"Advanced da Vinci surgical system simulator for surgeon training and operation planning", International Journal of Medical Robotics and Computer Assisted Surgery, Volume 3, No. 3, 19 June 2007, pages 245-251 discloses a two-handed interface to control a realistic model of a da Vinci robotic surgery system.

**Brief Summary of the Invention**

According to the present invention there is provided an apparatus and method as set forth in the appended claims. Other features of the invention will be apparent from the dependent claims, and the description which follows.

**Description of the Drawings**

For a fuller understanding of the nature and objects of the invention, reference should be made to the following detailed description taken in conjunction with the accompanying drawings, in which:

Figure 1A is a perspective view of a system according to an embodiment of the present invention;
Figure 1B is a perspective view of a system according to another embodiment of the present invention showing a binocular display;
Figure 2 is a perspective view of the system of Figure 1A being operated by a person;
Figure 3A is screen view of a display showing two virtual surgical tools;
Figure 3B is another screen view of a display showing two virtual surgical tools;
Figure 4 A is a line diagram of a dVSS manipulator;
Figure 4B is a line diagram of a RoSS input device;
Figure 5A is a diagram showing parameters in Denavit-Hartenberg notation;
Figure 5B is another diagram showing DH parameters in Denavit-Hartenberg notation;
Figure 6A is a graph showing the dVSS manipulator workspace map;
Figure 6B is a graph showing the RoSS input device workspace map;
Figure 6C is a graph showing the dVSS slave robot map;
Figure 6D is another graph showing the RoSS input device workspace map;
Figure 7A is a diagram of a dVSS slave robot;
Figure 7B is a diagram of the end-effector of a dVSS slave robot;

Figure 8A is a graph showing the intermediate configurations of three joints of the dVSS slave robot with inverse kinematics of a known RoSS input device position;

Figure 8B is a graph showing the intermediate positions of the dVSS slave robot with inverse kinematics of the same RoSS input device position as in Figure 8 A ;

Figure 9 is a graph showing the limits of the RoSS input device;

Figure 10A is a graph showing the intermediate configurations of three joints of the dVSS slave robot with inverse kinematics of a known RoSS input device position;

Figure 10B is a graph showing the intermediate positions of the dVSS slave robot with inverse kinematics of the same RoSS input device position as in Figure 10A; and

Figure 11 is a method according to another embodiment of the present invention.

## Detailed Description of the Invention

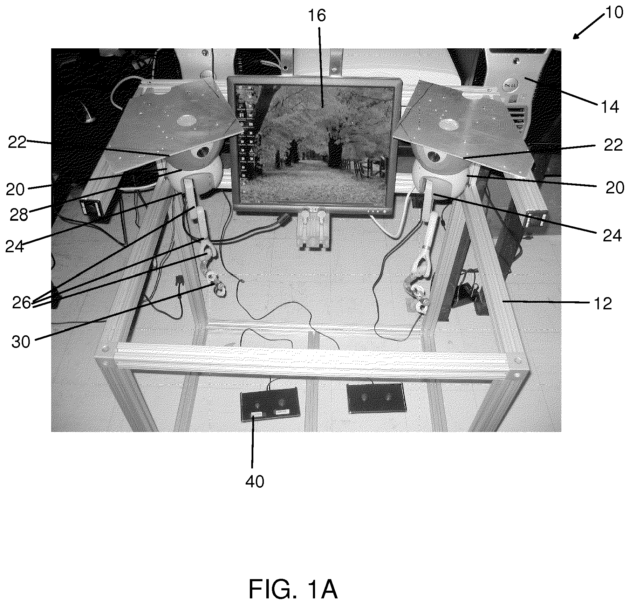

Figure 1A depicts a system 10 according to an embodiment of the invention which may include a frame 12, a computer 14, a display 16, and two input devices 20 each having an end-effector 30. The frame 12 may be adjustable so that it may be more comfortable for an operator or so that it more accurately replicates the configuration of an actual surgical robot master console. The frame 12 may be made from a lightweight material and designed to fold substantially flat for easier portability. The display 16 and computer 14 may be attached to the frame 12.

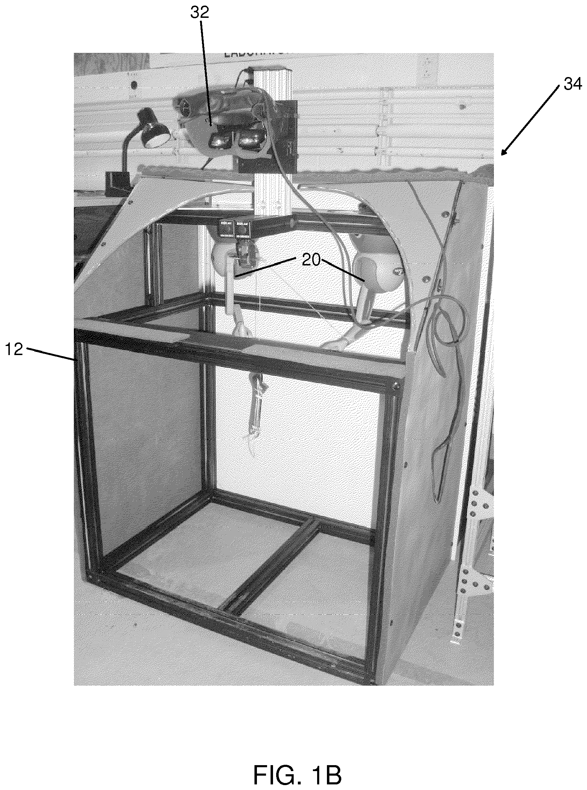

The display 16 may be in communication with the computer 14. In this way, the computer 14 may provide the data shown on the display 16. The display 16 may comprise a second display. In this way, a stereoscopic image may be displayed to provide 3 dimensional ("3D") viewing. The display 16 may be a binocular display 32; Figure 1B shows one such binocular display 32 which may be used in a system 34 according the invention. The display 16 may be affixed to the frame 12 or worn by a user (e.g. head-mounted). The display 16 may be a touchscreen or may further comprise a second display which may be a touchscreen display for controlling the computer 14.

The input devices 20 may be articulated armature devices suitable for providing 3D input. Each input device 20 may comprise a base 22, an arm 24, a plurality of joints 26, and an end-effector 30. The input devices 20 may provide at least six degrees of freedom. For example, a PHANTOM Omni® device from Sensable Technologies, Inc. may be used. While the Omni® device is depicted in the drawings, any 2 link mechanism having one free rotation at the base 22, two rotations at the links and three rotations of the wrist can be used. Two input devices 20 may be used to simulate the master console of a surgical robot. The input devices 20 may be attached to the frame 12 in an "upside-down" configuration wherein the base 22 of each input device 20 is affixed to the frame 12 and the first joint 28 of the arm 24 is positioned below the base 22. The end-effector of the input device may comprise a pinch input to provide a seventh degree of freedom. The pinch input may cause a clasping motion of the jaws of a virtual surgical tool. The input devices 20 may be in communication with the computer 14, and each input device 20 may provide a position signal corresponding to a position of the arm 24 of the input device 20 to the computer 14. The position of the arm 24 may be provided by sensing the position of the joints 26.

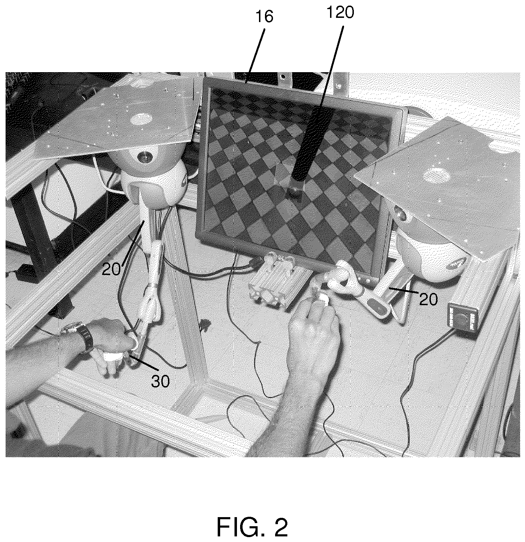

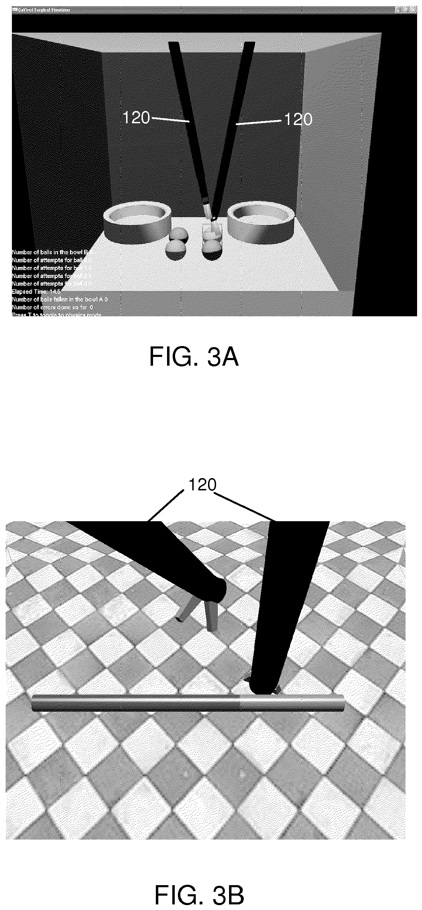

The computer 14 may be programmed to provide an image of one or more virtual surgical tools 120 to the display 16 (see, e.g., Figures 2, 3A, and 3B). The computer 14 may be additionally programmed to accept a position signal from at least one of the input devices 20 and transform the position signal into a position of the virtual surgical tool 120 depicted on the display 16. In this manner, changes in the position of at least one of the input devices 20 will be reflected as similar changes in the position of a virtual surgical tool 120 depicted on the display 16. The computer 14 may be programmed to use a mathematical transform function to alter the relationship between movement of the input device 20 in real space and movement of the virtual surgical tool 120 in virtual space. In this way, the virtual surgical tool 120 may move, for example, twice as fast as the input device 20, half as fast as the input device 20, the virtual surgical tool 120 may filter out a high frequency signal in the input (tremor), or any other relationship may be mapped.

The system 10 may also include a foot-operated input 40 such as, for example, a foot pedal. The foot-operated input 40 may provide functions such as engaging/disengaging tools, cauterization, cutting, turn lights on or off, changing a camera position, etc. More than one foot-operated input 40 may be used. The foot-operated input 40 may also enable additional virtual surgical tools 120 to be controlled by the input devices 20. For example, a three virtual surgical tools 120 may be controlled by two input devices 20 by using a foot-operated input 40 to selectively change the virtual surgical tools being controlled at any given time.

The computer 14 may be programmed with a Jacobian transform function wherein the Jacobian transform is calculated to substantially mimic the relationship of a position of a manipulator to a position of a slave robot in a surgical robot. In this manner, the system 10 may be programmed to replicate the kinematics of a particular surgical robot, such as, for example, a dVSS. As such, the system 10 may easily be reconfigured to mimic the feel of various surgical robots by providing a particular Jacobian transform to mimic the desired surgical robot.

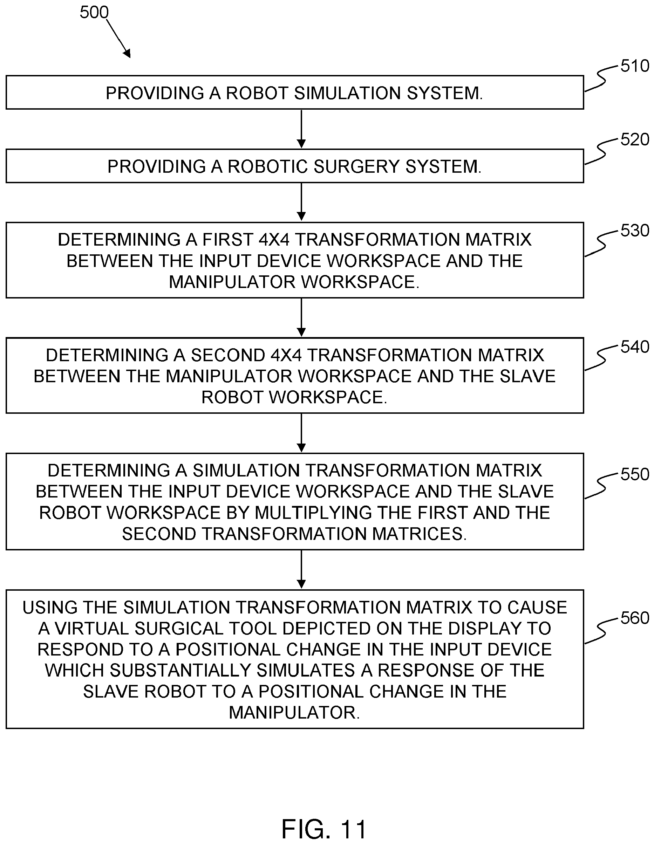

The present invention may also be embodied as a method for simulating the kinematics of a robotic surgery system 500 (see, e.g., Figure 11). Such a method 500 may comprise the steps of providing 510 a robot simulation system. The robot simulation system may comprise a computer, a display in communication with the computer, and an input device in communication with the computer. The robot simulation system may be similar to that described above. The input device may have an input device workspace which is defined by the range of motion of the input device. The method 500 may further comprise the step of providing 520 a robotic surgery system, such as, for example, a dVSS. The robotic surgery system may comprise a master console having a manipulator. The manipulator may have a manipulator workspace defined by the range of motion of the manipulator. The robotic surgery system may further comprise a slave robot having a slave robot workspace defined by the range of motion of the slave robot.

A first 4X4 transformation matrix may be determined 530, the first 4X4 transformation matrix relating the input device workspace and the manipulator workspace. A second 4X4 transformation matrix may be determined 540, the second 4X4 transformation matrix relating the manipulator workspace and the slave workspace. A simulation transformation matrix may be determined 550 by multiplying the first 4X4 transformation matrix and the second 4X4 transformation matrix. The simulation transformation matrix may relate the input device workspace and the slave robot workspace. The simulation transformation matrix may be used 560 to cause a virtual surgical tool depicted on the display of the robot simulation system to respond to a positional change in the input device which substantially simulates a response of the slave robot to a positional change in the manipulator. In this way, the kinematics of the input device as related to the virtual surgical tool may substantially simulate the kinematics of the manipulator as related to the slave robot.

The invention is further described through example 1 below.

Example 1

In the following non-limiting example, a dVSS was simulated by a robotic surgical simulator (RoSS) system embodying the current invention.

Kinesthetic Mapping

Kinesthetic mapping may be used to determine the transformation matrix between the simulator console workspace and the virtual slave workspace. This would help in transforming the RoSS input device positions to the virtual surgical tool positions which in turn will result in the virtual surgical tool motion. It may be done in two parts: (1) the workspace mapping of the simulator console and the manipulator of the surgical robot master console, and (2) the workspace mapping of the manipulator of the surgical robot master console and the surgical robot slave.

Workspace Mapping of RoSS Input Device and dVSS Manipulator using Forward Kinematics

Forward kinematics may be used to calculate the end-effector position and orientation given the angles of all the robot joints. Sensors may be located at the joints to measure the joint angles. This may result in a unique end-effector solution in 3 dimensional space. Matrix algebra is used to describe three dimensional rotations and translations. The complexity of expressions grows exponentially with number of joints (degrees of freedom).

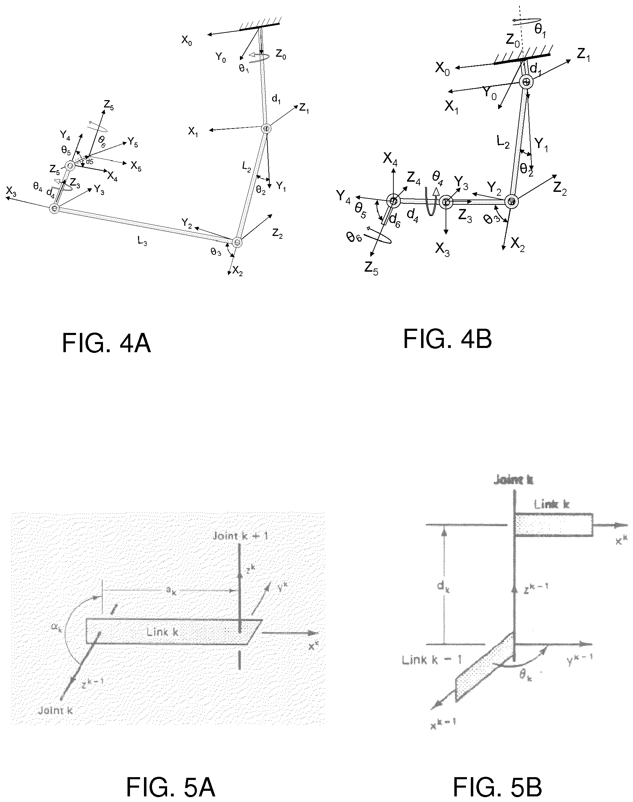

Figure 4A depicts a line diagram of the manipulator of a dVSS master console; Figure 4B depicts a line diagram of the RoSS input device. The dVSS manipulator may be viewed as an arm and a wrist. The arm of the dVSS manipulator may have three degrees of freedom and comprise a shoulder and an elbow-the shoulder having two degrees of freedom, and the elbow having one degree of freedom. The five degrees of freedom of the wrist of the dVSS manipulator were collapsed and mapped to the three degrees of freedom of the RoSS input device. Due to the redundant degrees of freedom of the wrist of the manipulator, the 5 degrees of freedom could be collapsed to 3 degrees of freedom. The roll motion of the wrist of the manipulator was mapped to the roll motion of the wrist of the RoSS input device. The yaw motion of the jaws of the wrist of the manipulator was mapped to the yaw motion of the end-effector of the RoSS input device and the clasping of jaws was mapped to the clasping action of the pinch of the custom wrist of the RoSS input device.

Modified Denavit-Hartenberg ("DH") notation was used to kinematically model the manipulator and the input device. DH parameters were calculated for the dVSS manipulator and the RoSS input device. DH notation is a systematic notation for assigning orthonormal coordinate frames to the joints. The following steps were required in order to assign coordinate frames to the joints:

(1) Assign a coordinate frame $L_0$ to the dVSS manipulator base;
(2) Align $z_k$ with the axis of joint k + 1;
(3) Locate the origin of $L_k$ at the intersection of $z_k$ and $z_{k-1}$. When there is no intersection, use the intersection of $z_k$ with a common normal between $z_k$ and $z_{k-1}$;
(4) Select $x_k$ to be orthogonal to $z_k$ and $z_{k-1}$. If $z_k$ and $z_{k-1}$ are parallel, point $x_k$ away from $z_{k-1}$; and
(5) Select $y_k$ to form a right handed orthonormal coordinate frame;

After assigning coordinate frames, the DH parameters may be calculated based on the following conventions (see Figures 5A and 5B):

(1) $\theta_k$ is the angle of rotation from $x_{k-1}$ to $x_k$ measured about $z_{k-1}$;

(2) $d_k$ is the distance measured along zk-1;

(3) $a_k$ is the distance measured along $x_k$; and

(4) $\alpha_k$ is the angle of rotation from $z_{k-1}$ to $z_k$ about $x_k$.

Each homogeneous transformation *T* may be represented as a product of four basic transformations associated with joints *i* and *j* (*l*-link length, $\alpha$-link twist, *d*-link offset, and $\theta$-joint angle) and *I* is a 4x4 identity matrix. The position and orientation of the end-effector was denoted by a position vector *P* and the 3x3 rotation matrix *R*. Based on the above DH parameters, a homogeneous transformation matrix was constructed which maps frame *i* coordinates into *i*-1 coordinates as follows:

$$T_{i-1}^{i} = \begin{bmatrix} \cos\theta_i & -\sin\alpha_i \cos\theta_i & \sin\alpha_i \sin\theta_i & a_i \cos\theta_i \\ \sin\theta_i & -\cos\alpha_i \cos\theta_i & \sin\alpha_i \cos\theta_i & a_i \sin\theta_i \\ 0 & \sin\alpha_i & \cos\alpha_i & d_i \\ 0 & 0 & 0 & 1 \end{bmatrix} \dots\dots\dots (1)$$

$$T_{i-1}^{i} = \begin{bmatrix} R & P \\ 0 \quad 0 \quad 0 & 1 \end{bmatrix} \dots\dots\dots\dots\dots\dots\dots\dots\dots\dots\dots\dots\dots (2)$$

After calculating the homogeneous transformation matrix for each link, the composite transformation matrix was calculated. This matrix maps the tool coordinates to the base coordinates. This yields the transformation matrix as:

$$T_{base}^{tool} = T_{base}^{wrist} \times T_{wrist}^{tool} \dots\dots\dots\dots\dots\dots\dots\dots\dots\dots (3)$$

This final composite transformation matrix was calculated with respect to the base frame. The DH parameters for the dVSS manipulator are shown in Table 1.

Table 1: DH Parameters of dVSS Manipulator

| Link | Parameters | $\theta$ | d | a | $\alpha$ |
|------|-----------|----------|----|----|----------|
| 1 | $\theta1$ | $\theta1$ | d1 | 0 | -pi/2 |
| 2 | $\theta2$ | $\theta2$ | 0 | L2 | 0 |
| 3 | $\theta3$ | $\theta3$ | 0 | L3 | -pi/2 |
| 4 | $\theta4$ | $\theta4$ | d4 | 0 | pi/2 |
| 5 | $\theta5$ | $\theta5$ | d5 | 0 | -pi/2 |
| 6 | $\theta6$ | $\theta6$ | d6 | 0 | pi/2 |

The DH parameters for the RoSS input device are shown in Table 2.

Table 2 : DH Parameters of RoSS Input Device

| Link | Parameters | $\theta$ | d | a | $\alpha$ |
|------|-----------|----------|-----|-----|-------|
| 1 | $\theta 1$ | $\theta 1$ | d1 | 0 | -pi/2 |
| 2 | $\theta 2$ | $\theta 2$ | 0 | L2 | 0 |
| 3 | $\theta 3$ | $\theta 3$ | 0 | 0 | -pi/2 |
| 4 | $\theta 4$ | $\theta 4$ | d4 | 0 | pi/2 |
| 5 | $\theta 5$ | $\theta 5$ | 0 | 0 | -pi/2 |
| 6 | $\theta 6$ | $\theta 6$ | d6 | 0 | pi/2 |

Based on these DH parameters, the individual transformation matrix for each link may be calculated and the composite transformation matrix may be constructed after multiplying each of the individual transformation matrices as follows

$$T_0^6 = T_0^1 \times T_1^2 \times T_2^3 \times T_3^4 \times T_4^5 \times T_5^6 \dots\dots\dots\dots\dots\dots\dots\dots\dots\dots \quad (4)$$

To find the overall workspaces of the RoSS input device and dVSS manipulator, the range of angles of all the joints was found.

The range of each of the joint angles of RoSS input device was:

Joint 1:     $-1.45 < \theta_1 < 1.05$ (radians)
Joint 2:     $0.0 < \theta_2 < 1.727$ (radians)
Joint 3:     $1.0 < \theta_3 < 2.1$ (radians)
Joint 4:     $0.0 < \theta_4 < 4.71$ (radians)
Joint 5:     $0.0 < \theta_5 < 3.0$ (radians)
Joint 6:     $0.0 < \theta_6 < 4.71$ (radians)

The range of each of the joint angles of dVSS manipulator was:

Joint 1:     $-0.53 < \theta_1 < 1.57$ (radians)
Joint 2:     $0.265 < \theta_2 < 0.785$ (radians)
Joint 3:     $0.0 < \theta_3 < 1.03$ (radians)
Joint 4:     $-3.14 < \theta_4 < 1.57$ (radians)
Joint 5:     $-1.57 < \theta_5 < 3.14$ (radians)
Joint 6:     $-0.707 < \theta_6 < 0.707$ (radians)

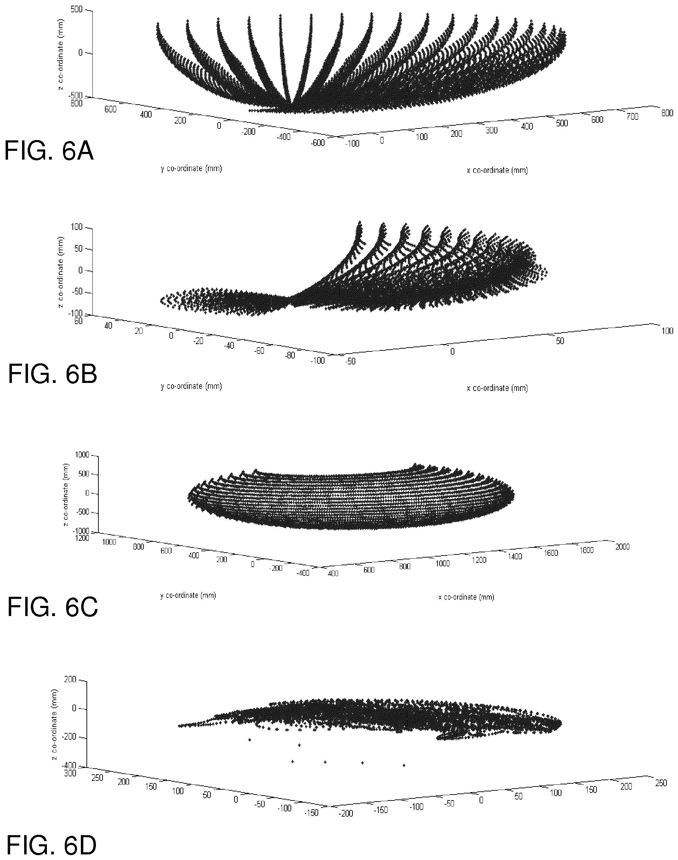

Each of the joint angles was varied incrementally to yield the end-effector positions in the workspace (Figure 6A and 6B). The end-effector position matrix was homogenized by adding a fourth column to the x, y and z columns. The workspace positions for both the RoSS input device and the dVSS manipulator were calculated. The 4X4 transformation matrix between the two workspaces was calculated by:

$$T = pinv(P_O) * P_M \dots\dots\dots\dots\dots\dots\dots\dots\dots\dots\dots\dots\dots\dots\dots\dots\dots\dots\dots \quad (5)$$

where:

P$_O$ is the set of homogenized positions for RoSS input device; and
P$_M$ is the set of homogenized positions for dVSS manipulator.

Since the end-effector encoder position values from the RoSS input device were spatially transformed to the calculated position values of RoSS input device from DH notation, these positions may either be transformed to the RoSS input device workspace or transformed to the dVSS manipulator workspace. Therefore, a set of device positions consisting of a large number of 3D spatial position values (9261 in number) and the end-effector positions were homogenized by adding a fourth column to x, y and z columns. Then the 4X4 transformation matrix was found between the two workspaces.

Workspace Mapping of dVSS Master Console and dVSS Slave Robot

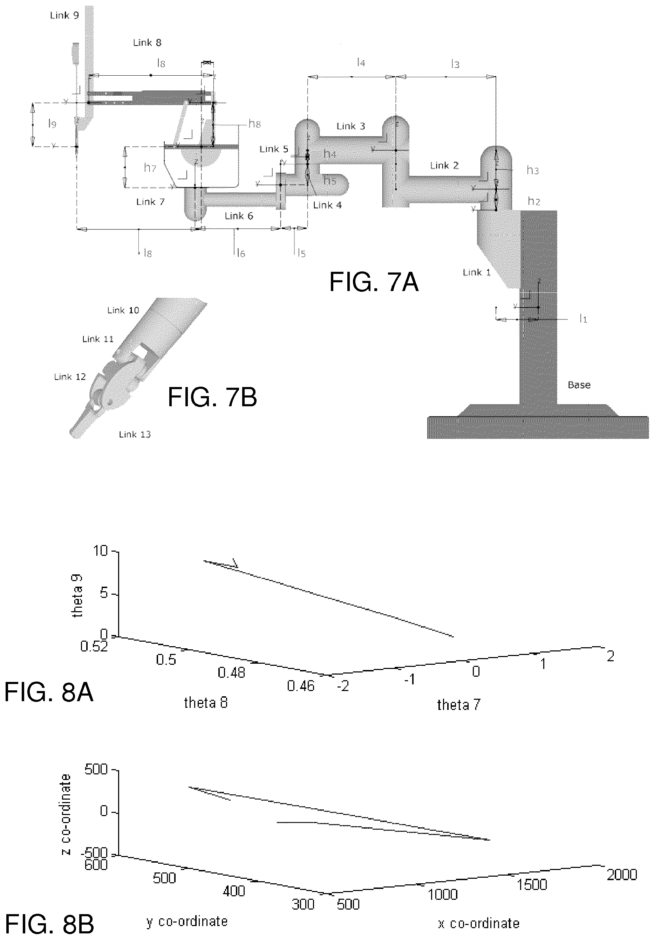

The manipulator of the dVSS controls a highly articulated laparoscopic tool of the dVSS slave robot shown in Figures 7A and 7B, the end-effector of which has seven degrees of freedom. In the RoSS console, the three degrees of freedom of the shoulder and elbow of the input devices were mapped such that the virtual surgical tool is pivoted about the site of entry and has two rotational degrees of freedom and one translational degree of freedom along its axis. Two of the three degrees of freedom of the wrist were mapped to the jaws to allow rotational movement of the virtual surgical tool and the clasping of the jaws was mapped to a clasping action of the pinch of the custom wrist. The virtual surgical tool has the same degrees of freedom as the tool which is used with the dVSS. In order to map the RoSS input device with the virtual surgical tool, the dVSS manipulator was mapped to the dVSS slave robot.

Modified Denavit-Hartenberg ("DH") notation was again used to kinematically model the dVSS slave robot. The corresponding controlled parameters of the arm are summarized in Table 3. The kinematic equations of the arm for the instrument and the endoscope are represented by a total of 12 degrees of freedom (2 translational and 10 rotational).

Table 3 DH Parameters of dVSS Slave Robot

| Link | Parameters | $\theta$ | d | a | $\alpha$ |
|---|---|---|---|---|---|
| 1 | t1 | 0 | t1 | L1 | 0 |
| 2 | $\theta2$ | $\theta2$ | H2 | 0 | 0 |
| 3 | $\theta3$ | $\theta3$ | H3 | L3 | 0 |
| 4 | $\theta4$ | $\theta4$ | H4 | L4 | 0 |
| 5 | $\theta5$ | $\theta5$ | H5 | L5 | -pi/2 |
| 6 | $\theta6$ | $\theta6$ | 0 | L6 | pi/2 |
| 7 | $\theta7$ | $\theta7$ | H7 | 0 | -pi/2 |
| 8 | $\theta8$ | $\theta8$ | H8 | L8 | 0 |
| 9 | t9 | 0 | t9 | L9 | 0 |
| 10 | $\theta10$ | $\theta10$ | 0 | 0 | 0 |
| 11 | $\theta11$ | $\theta11$ | L11 | 0 | -pi/2 |
| 12 | $\theta12$ | $\theta12$ | 0 | L12 | pi/2 |

The corresponding transformation matrices between links 1 to 6, links 7 to 9, and links 10 to 12 are $T_0^6$, $T_7^9$ and $T_{10}^{12}$. Links 1 to 6 correspond to the robot base, links 7 to 9 to the robot mechanism, and links 10 to 12 to the instruments. The robot base includes five revolute joints and one prismatic joint. A double parallelogram linkage mechanism is formed on link 8. The robot arm of the mechanism has three degrees of freedom, with two revolute joints and a prismatic joint.

Based on these DH parameters, the individual transformation matrix for each link was calculated as described above and the composite transformation matrix between the slave robot base and the end-effector of the slave robot (instrument) was constructed after multiplying each of the individual transformation matrices as follows

$$T_0^{12} = T_0^1 \times T_1^2 \times T_2^3 \times T_3^4 \times T_4^5 \times T_5^6 \times T_6^7 \times T_7^8 \times T_8^9 \times T_9^{10} \times T_{10}^{11} \times T_{11}^{12} \dots (6)$$

Table 4: DH Parameters of dVSS Manipulator

| Link | Parameters | $\theta$ | d | a | $\alpha$ |
|---|---|---|---|---|---|
| 1 | $\theta1$ | $\theta1$ | d1 | 0 | -pi/2 |
| 2 | $\theta2$ | $\theta2$ | 0 | L2 | 0 |

(continued)

| Link | Parameters | $\theta$ | d | a | $\alpha$ |
|------|-----------|----------|-----|-----|----------|
| 3 | $\theta 3$ | $\theta 3$ | 0 | L3 | -pi/2 |
| 4 | $\theta 4$ | $\theta 4$ | d4 | 0 | pi/2 |
| 5 | $\theta 5$ | $\theta 5$ | 0 | L5 | -pi/2 |
| 6 | $\theta 6$ | $\theta 6$ | d6 | 0 | pi/2 |

The first six degrees of freedom of dVSS slave robot were initially set to a particular configuration. The next three degrees of freedom of dVSS slave robot (two revolute and one prismatic joint) were mapped to first three degrees of freedom (three revolute joints) of the manipulator. The last three degrees of freedom of dVSS slave robot and dVSS manipulator were also set to a particular configuration.

To find the overall workspaces of dVSS manipulator and dVSS slave robot, the range of joint angles of all the slave robot links was found. The range of each of the joint angles of dVSS slave robot was as follows:

Joint 1: $t_1 = 23$ cm (9.0 inches)
Joint 2: $\theta_2 = 0.0$ (radians)
Joint 3: $\theta_3 = 0.0$ (radians)
Joint 4: $\theta_4 = 0$ (radians)
Joint 5: $\theta_5 = 1.05$ (radians)
Joint 6: $\theta_6 = 1.05$ (radians)
Joint 7: $-1.57 < \theta_7 < 1.57$ (radians)
Joint 8: $-1.05 < \theta_8 < 1.05$ (radians)
Joint 9: $0.0 < t_9 < 23$ cm (9.0 inches)
Joint 10: $0.0 < \theta_{10} < 7.33$ (radians)
Joint 11: $0.0 < \theta_{11} < 3.14$ (radians)
Joint 12: $0.0 < \theta_{12} < 3.14$ (radians)

The range of each of the joint angles of the dVSS manipulator was:

Joint 1: $-0.53 < \theta_1 < 1.57$ (radians)
Joint 2: $0.265 < \theta_2 < 0.785$ (radians)
Joint 3: $0.0 < \theta_3 < 1.03$ (radians)
Joint 4: $-3.14 < \theta_4 < 1.57$ (radians)
Joint 5: $-1.57 < \theta_5 < 3.14$ (radians)
Joint 6: $-0.707 < \theta_6 < 0.707$ (radians)

Each of the joint angles was varied incrementally to yield the end-effector positions in the workspace. Then the end-effector positions were homogenized by adding a fourth column to x, y and z columns. The workspace positions for both the dVSS manipulator (Figure 6A) and dVSS slave robot (Figure 6C) was calculated. The 4X4 transformation matrix between the two workspaces was calculated by

$$T = pinv(P_M) * P_S \dots\dots\dots\dots\dots\dots\dots\dots\dots\dots\dots\dots\dots\dots\dots\dots\dots\dots\dots\dots\dots\dots\dots (7)$$

where:

$P_S$ is the set of homogenized positions for dVSS slave robot; and
$P_M$ is the set of homogenized positions for dVSS manipulator.

Inverse Kinematics Using Jacobian-Based Control

Inverse kinematics may be used to find a set of joint configurations of an articulated structure based upon a desirable end-effector location. Inverse kinematics was used to determine a set of joint angles in an articulated structure based upon the position of the end-effector of the slave robot. This results in multiple joint angle solutions and infinite solutions at singularities. It may be generally used in software to control the joints. Control software should be able to perform the necessary calculations in near real time.

The mathematical representation of the inverse kinematics technique is defined as

$$\theta = f^{-1}(X) \dots\dots\dots\dots\dots\dots\dots\dots\dots\dots\dots\dots\dots (8)$$

Inverse kinematics may be implemented based upon the Jacobian technique. This technique incrementally changes joint orientations from a stable starting position towards a joint configuration that will result in the end-effector being located at the desired position in absolute space. The amount of incremental change on each iteration is defined by the relationship between the partial derivatives of the joint angles, $\theta$, and the difference between the current location of the end-effector, $X$, and the desired position, $X_d$. The link between these two sets of parameters leads to the system Jacobian, J. This is a matrix that has dimensionality *(m* x *n)* where *m* is the spatial dimensional of *X* and *n* is the size of the joint orientation set, q.

$$X = f(\theta) \dots\dots\dots\dots\dots\dots\dots\dots\dots\dots\dots\dots\dots\dots\dots (9)$$

The Jacobian was derived from Equation 9 as follows. Taking partial derivatives of Equation 9:

$$dX = J(\theta)d\theta \dots\dots\dots\dots\dots\dots\dots\dots\dots\dots\dots\dots\dots (10)$$

Where:

$$J_{ij} = \frac{\partial f_i}{\partial \theta_j} \dots\dots\dots\dots\dots\dots\dots\dots\dots\dots\dots\dots\dots (11)$$

Rewriting Equation 10 in a form similar to inverse kinematics (Equation 9), results in Equation 12. This form of the problem transforms the under-defined system into a linear one that can be solved using iterative steps.

$$d\theta = J^{-1}dX \dots\dots\dots\dots\dots\dots\dots\dots\dots\dots\dots\dots\dots (12)$$

The problem now is that Equation 12 requires the inversion of the Jacobian matrix. However because of the under-defined problem that the inverse kinematics technique suffers from, the Jacobian is very rarely square. Therefore, the right-hand generalized pseudo-inverse may be used to overcome the non-square matrix problem, as given in equation 14.

Generating the pseudo-inverse of the Jacobian in this way can lead to inaccuracies in the resulting inverse that need to be reduced. Any inaccuracies of the inverse Jacobian can be detected by multiplying it with the original Jacobian then subtracting the result from the identity matrix. A magnitude error can be determined by taking the second norm of the resulting matrix multiplied by *dP*, as outlined in Equation 15. If the error proves too big then *dP* can be decreased until the error falls within an acceptable limit.

An overview of the algorithm used to implement an iterative inverse kinematics solution is as follows:

(1) Calculate the difference between the goal position and the actual position of the end-effector.

$$dP = X_g - X_p \dots\dots\dots\dots\dots\dots\dots\dots\dots\dots\dots\dots\dots (13)$$

(2) Calculate the Jacobian matrix using the current joint angles.

$$J_{ij} = \frac{\partial P_i}{\partial \theta_j} \quad\text{......................................................................} \quad (14)$$

(3) Calculate the pseudo-inverse of the Jacobian.

$$J^{-1} = J^{T}\left(JJ^{T}\right)^{-1} \quad\text{.......................................................} \quad (15)$$

(4) Determine the error of the pseudo-inverse error:

$$\text{error} = \left\| I - \left(JJ^{-1}\right)\!dP \right\| \quad\text{..............................................} \quad (16)$$

(5) If error > e then dP = dP / 2 restart at step (4)
(6) Calculate the updated values for the joint orientations and use these as the new current values. Check the bounds for theta values.

$$\theta = \begin{cases} \text{lowerbound if } \theta + J^{-1}dP < \text{lowerbound} \\ \text{upperbound if } \theta + J^{-1}dP > \text{upperbound} \\ \quad\quad \theta + J^{-1}dP \text{ if otherwise} \end{cases} \quad\text{.................} \quad (17)$$

(7) Using forward kinematics determine whether the new joint orientations position the end-effector close enough to the desired absolute location. If the solution is adequate then terminate the algorithm otherwise go back to step (1).

The time to complete the Inverse Kinematics algorithm for a given end-effector is an unknown quantity due to an arbitrary number of iterations required. However, the time to complete a single iteration is constant with respect to the dimensionality of $X$ and $\theta$ which is unchanged under a complete execution of the algorithm. Therefore by placing an upper limit on the number of iterations we can set a maximum time boundary for the algorithm to return in. If the solver reaches the limit then the algorithm returns the closest result it has seen.

Jacobian-Based Inverse Kinematics Applied to Simulation

The dVSS slave robot consists of 12 degrees of freedom and the inverse kinematics were used to control only the three degrees of freedom. The first six degrees of freedom were fixed to suitable joint angles. These joint angles were calculated on the basis of a particular dVSS configuration during a surgical procedure in the operating room. The next three degrees of freedom are being considered for inverse kinematics control. These were two revolute joints and one prismatic joint. The last three degrees of freedom were also constrained as part of wrist at a particular configuration, (pi/2, pi/2, 0 radians).
The algorithm requires that the three joint angles which correspond to a particular configuration of dVSS are initialized. Then using forward kinematics, the end-effector position of the dVSS surgical tool (slave robot) was found which correspond to the initial position of the slave robot. Once the initial position is found the difference between the goal and initial position was calculated. The Jacobian was calculated by differentiating the end-effector position with respect to the three slave robot joint angles (two revolute joints and one prismatic joint) using the following equation:

$$J = \begin{bmatrix} dPx/d\theta_7 & dPx/d\theta_8 & dPx/dt_9 \\ dPy/d\theta_7 & dPy/d\theta_8 & dPy/dt_9 \\ dPz/d\theta_7 & dPz/d\theta_8 & dPz/dt_9 \end{bmatrix} \quad\text{...............................} \quad (18)$$

Once the Jacobian was known, its pseudo inverse was calculated. The pseudo inverse was used to reduce the singularities in the matrix. Then the three joint angles are updated using the following equation:

$$\begin{bmatrix} \theta_7 \\ \theta_8 \\ t_9 \end{bmatrix} = \begin{cases} \text{lowerbound if} \begin{bmatrix} \theta_7 \\ \theta_8 \\ t_9 \end{bmatrix} + J^{-1} \begin{bmatrix} dP_x \\ dP_y \\ dP_z \end{bmatrix} < \text{lowerbound} \\ \text{upperbound if} \begin{bmatrix} \theta_7 \\ \theta_8 \\ t_9 \end{bmatrix} + J^{-1} \begin{bmatrix} dP_x \\ dP_y \\ dP_z \end{bmatrix} > \text{upperbound} \dots\dots\dots\dots (19) \\ \begin{bmatrix} \theta_7 \\ \theta_8 \\ t_9 \end{bmatrix} + J^{-1} \begin{bmatrix} dP_x \\ dP_y \\ dP_z \end{bmatrix} \text{ if otherwise} \end{cases}$$

Using forward kinematics equations, the three joint orientations are used to calculate the end-effector position and the difference between the goal position and the current end-effector position was checked. If the two were close enough then the algorithm is terminated.

Figures 8A and 8B shows a complete cycle of an inverse kinematics algorithm for a particular RoSS input device position. The initial input device position was [-24.7935,-47.8608,-47.7267] mm and when it was transformed to the dVSS slave robot position which was the goal position. The initial dVSS slave robot position was calculated using the initial joint configuration of the dVSS slave robot (using DH notation) where the joint values of the 7th, 8th, and 9th joint are taken as -pi/6 radians, pi/4 radians, and 25mm (1 inch) respectively. The algorithm took 8 iterations and the intermediate joint configuration and position values are shown in Figures 8A and 8B.

The motions of the virtual surgical tool were performed using inverse kinematics which was essentially controlled by the RoSS input device. The input device positions were transformed to the positions of the virtual surgical tool and then the inverse kinematics was performed to calculate the link parameters (joint angles) of the virtual surgical tool.

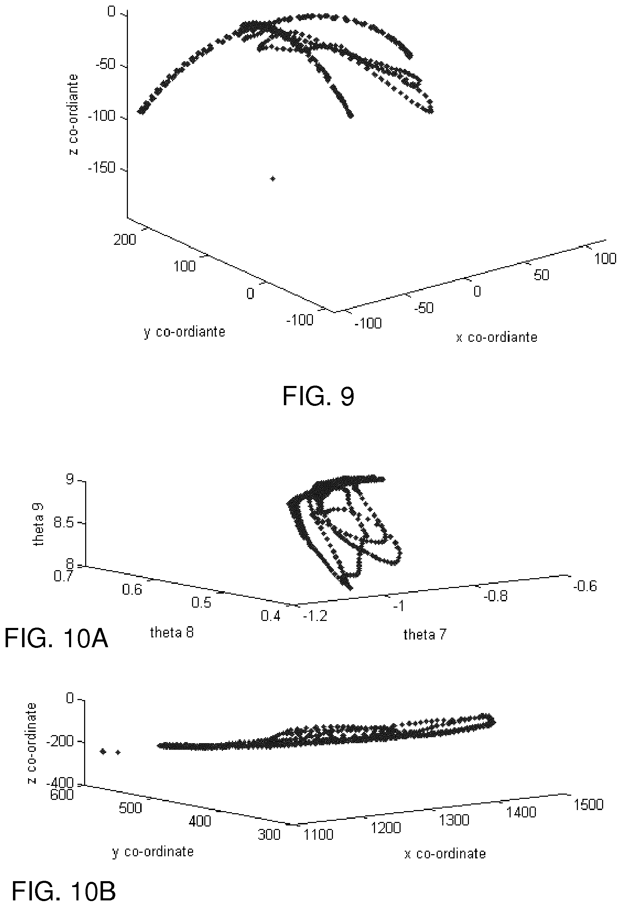

Figure 9 is a graph of RoSS input device positions. The positions were homogenized. The transformation matrix between the RoSS input device positions and dVSS manipulator workspace was calculated by taking the pseudo inverse of device positions and multiplying by dVSS manipulator homogeneous positions using Equation 5 (as described above).

Similarly the transformation matrix between dVSS manipulator and dVSS slave robot was obtained as described above.

The transformation matrix between the RoSS input device workspace and dVSS slave robot workspace was found by multiplying the two transformation matrices. Figures 6C depicts the workspace mapping of the dVSS slave robot and Figure 6D depict the workspace mapping of the RoSS input device (using device positions).

Once the transformation matrix was obtained, the RoSS input device position was transformed to the dVSS slave robot position. After the dVSS slave robot position was obtained, the inverse kinematics was performed to get the joint angles of 7th, 8th and 9th links as described above. The 7th and 8th links are revolute joints and 9th link is a prismatic joint. The accuracy of the algorithm is $10^{-5}$ mm and the computational time is 25-35 ms for computing each of the three sets of joint angles using inverse kinematics algorithm. The number of iterations was set to 40 for a single run of algorithm. The motion of the virtual surgical tool was manipulated to get the desired motion of dVSS slave robot.

Figure 10A shows joint configurations of the RoSS input device, and Figure 10B shows the workspace of the RoSS input device using inverse kinematics for a set of input device positions.

The bounds on the 7th, 8th and 9th Joint Configurations for the dVSS slave robot using inverse kinematics algorithm were found to be:

Joint 7:  $-1.15 < \theta_7 < -0.75$ (radians)
Joint 8:  $0.35 < \theta_8 < 0.60$ (radians)
Joint 9:  19 (7.5) $< t_9 <$ 23 (9.0) cm (inches)

These joint values were used to calculate the end-effector boundaries of the dVSS slave robot. The dVSS slave robot was treated as a six degrees of freedom robot (3 degrees of freedom for arm and 3 degrees of freedom for wrist) so that the axis orientations of the dVSS slave robot and the RoSS input device were coincident.

The workspace boundaries of the dVSS slave robot from the above joint range using DH notation was found to be:

$$170 < x < 370 \text{ mm}$$

$$-450 < y < -250 \text{ mm}$$

$$-120 < z < -5 \text{ mm}$$

The workspace boundaries of end-effector of the RoSS input device position were:

$$-108 < x < 117 \text{ mm}$$

$$-110 < y < 240 \text{ mm}$$

$$-200 < z < -10 \text{ mm}$$

Since the axis orientations of the dVSS slave robot and the RoSS input device are the same, the x, y, z axes of RoSS input device were mapped onto the x, y, z axes of dVSS slave robot. Then the two rotations along the x and z axes and one translation along the y axis were calculated to give the actual dVSS slave robot motions.

Although the present invention has been described with respect to one or more particular embodiments, it will be understood that other embodiments of the present invention may be made without departing from the scope of the present invention. Hence, the present invention is deemed limited only by the appended claims and the reasonable interpretation thereof.

**Claims**

**1.**    A surgical robot simulation system (10) comprising:

a frame (12);
a computer (14);
a display (16) in communication with the computer;
two input devices (20) in communication with the computer, each input device having a base (22), an arm (24), and an end-effector (30), wherein the arms comprise a plurality of joints (26), the input devices being attached to the frame so that a first joint of each arm, in use, resides beneath the base, and wherein each of the input devices provides a position signal to the computer, the positional signal corresponding to a position of the arm of the input device; and
wherein the computer is programmed to: accept a position signal from each of the input devices; and transform each of the position signals into a position of a virtual surgical tool depicted on the display using a simulation transformation matrix determined by:

determining a first 4X4 transformation matrix between a workspace of each input device workspace and a manipulator workspace;
determining a second 4X4 transformation matrix between the manipulator workspace and a slave robot workspace;
determining the simulation transformation matrix between the input device workspace and the slave robot matrix by multiplying the first and the second 4X4 transformation matrices.

**2.**    The surgical robot simulation system of claim 1 wherein the computer is further programmed to:
use a mathematical transform function to alter a relationship between movement of the arm in real space and movement of the virtual surgical tool in virtual space.

**3.**    The surgical robot simulation system of claim 2 wherein the mathematical transform function causes the relationship of arm and virtual surgical tool movements to substantially mimic the relationship of a position of a control to a position of a surgical tool in a surgical robot.

4. The surgical robot simulation system of claim 3 wherein the surgical robot is a da Vinci® surgical system.

5. The surgical robot simulation system of claim 1 wherein the display is a touchscreen.

6. The surgical robot simulation system of claim 1 further comprising a foot-operable input (40).

7. The surgical robot simulation system of claim 1 wherein the frame is adjustable.

8. The surgical robot simulation system of claim 7 wherein the frame may fold into a substantially flat shape.

9. The surgical robot simulation system of claim 1 wherein the display is a binocular display (32) capable of displaying a stereoscopic image.

10. The surgical robot simulation system of claim 1 wherein each input device provides at least six degrees of freedom.

11. A method for simulating the kinematics of a robotic surgery system comprising the steps of:

Providing (510) a robot simulation system comprising:

a computer;
a display in communication with the computer; and
an input device in communication with the computer, the input device having a input device workspace defined by the range of motion of the input device;

providing (520) a robotic surgery system comprising:

a master console having a manipulator, the manipulator having a manipulator workspace defined by the range of motion of the manipulator; and
a slave robot having a slave robot workspace defined by the range of motion of the slave robot;

determining (530) a first 4X4 transformation matrix between the input device workspace and the manipulator workspace;
determining (540) a second 4X4 transformation matrix between the manipulator workspace and the slave robot workspace;
determining (550) a simulation transformation matrix between the input device workspace and the slave robot matrix by multiplying the first and the second 4X4 transformation matrices; and
using (560) the simulation transformation matrix to cause a virtual surgical tool depicted on the display to respond to a positional change in the input device which substantially simulates a response of the slave robot to a positional change in the manipulator.

**Patentansprüche**

1. Ein chirurgisches Robotersimulationssystem (10), das Folgendes umfasst:

einen Rahmen (12); einen Computer (14);
einen Bildschirm (16), der mit dem Computer kommuniziert;
zwei Input-Geräte (20), die mit dem Computer kommunizieren, jedes Input-Gerät hat eine Basis (22), einen Arm (24) und einen End-Effektor (30), wobei die Arme mehrere Verbindungsstücke (26) haben und die Input-Geräte an dem Rahmen befestigt sind, sodass sich während des Betriebs ein erstes Verbindungsstück jedes Arms
unter der Basis befindet und jedes Input-Gerät ein Positionssignal an den
Computer sendet, welches einer Position des Arms des Input-Geräts entspricht; und wobei der Computer programmiert ist, um: ein Positionssignal von jedem der Input-Geräte zu akzeptieren; und jedes Positionssignal in eine Position eines virtuellen chirurgischen Tools zu verwandeln, das auf dem Bildschirm mit Hilfe einer Simulations-Verwandlungsmatrix abgebildet wird, die durch Folgendes bestimmt wird:

der Bestimmung einer ersten 4x4 Verwandlungsmatrix zwischen einem Arbeitsplatz von jedem Input-Gerät-Arbeitsplatz und einem Manipulator-Arbeitsplatz;
der Bestimmung einer zweiten 4x4 Verwandlungsmatrix zwischen dem Manipulator-Arbeitsplatz und einem Unterstützungs-Roboterarbeitsplatz;
der Bestimmung der Simulations-Verwandlungsmatrix zwischen dem Input-Gerät-Arbeitsplatz und der Unterstützungs-Robotermatrix, indem die erste und zweite 4x4 Verwandlungsmatrix multipliziert werden.

2. Das chirurgische Robotersimulationssystem aus Anspruch 1, wobei der Computer des Weiteren programmiert ist, um:

Eine mathematische Verwandlungsfunktion zu verwenden, um ein Verhältnis zwischen der Bewegung des Arms im echten Raum und der Bewegung des virtuellen chirurgischen Tools im virtuellen Raum zu verändern.

3. Das chirurgische Robotersimulationssystem aus Anspruch 2, wobei die mathematische Verwandlungsfunktion das Verhältnis des Arms und der Bewegungen des virtuellen chirurgischen Tools bewirkt, um das Verhältnis einer Position einer Steuereinheit und einer Position des chirurgischen Tools bei einem chirurgischen Roboter zu imitieren.

4. Das chirurgische Robotersimulationssystem aus Anspruch 3, wobei der chirurgische Roboter ein da Vincio chirurgisches System ist.

5. Das chirurgische Robotersimulationssystem aus Anspruch 1, wobei der Bildschirm ein Touchscreen ist.

6. Das chirurgische Robotersimulationssystem aus Anspruch 1, das des Weiteren einen mit dem Fuß zu bedienenden Input (40) umfasst.

7. Das chirurgische Robotersimulationssystem aus Anspruch 1, wobei der Rahmen anpassbar ist.

8. Das chirurgische Robotersimulationssystem aus Anspruch 7, wobei sich der Rahmen in eine solide flache Form falten lässt.

9. Das chirurgische Robotersimulationssystem aus Anspruch 1, wobei der Bildschirm ein binokularer Bildschirm ist, der ein stereoskopisches Bild anzeigen kann.

10. Das chirurgische Robotersimulationssystem aus Anspruch 1, wobei jedes Input-Gerät mindestens sechs Grad Freiräume bietet.

11. Eine Methode zur Simulation der Kinematik eines chirurgischen Robotersystems, die die folgenden Schritte umfasst:
Das Bereitstellen (510) eines Robotersimulationssystems, welches Folgendes umfasst:

einen Computer;
einen Bildschirm, der mit dem Computer kommuniziert; und
ein Input-Gerät, das mit dem Computer kommuniziert und einen Input-Gerät-Arbeitsplatz hat, der durch den Bewegungsbereich des Input-Geräts definiert ist;
Das Bereitstellen (520) eines Robotersimulationssystems, welches Folgendes umfasst:

eine Master-Konsole mit einem Manipulator, der einen Manipulator-Arbeitsplatz hat, welcher durch den Bewegungsbereich des Manipulators bestimmt ist; und
einen Unterstützungsroboter mit einem Unterstützungsroboter-Arbeitsplatz, der durch den Bewegungsbereich des
Unterstützungsroboters definiert ist;
die Bestimmung (530) einer ersten 4x4 Verwandlungsmatrix zwischen einem Input-Gerät-Arbeitsplatz und dem Manipulator-Arbeitsplatz;
die Bestimmung (540) einer zweiten 4x4 Verwandlungsmatrix zwischen dem Manipulator-Arbeitsplatz und dem Unterstützungs-Roboterarbeitsplatz;
die Bestimmung (550) einer Simulations-Verwandlungsmatrix zwischen dem Input-Gerät-Arbeitsplatz und der Unterstützungsroboter-Matrix, indem die erste und zweite 4x4
Verwandlungsmatrix multipliziert werden; und
die Verwendung (560) der Simulations-Verwandlungsmatrix, damit ein virtuelles chirurgisches Tool, das auf dem Bildschirm abgebildet ist, auf eine Positionsänderung in dem Input-Gerät, welches eine Reaktion des Unterstützungsroboters auf eine Positionsänderung in dem Manipulator simuliert, reagiert.

**Revendications**

1. Un système de simulation de robot chirurgical (10) incluant :

   un châssis (12) ;
   un ordinateur (14) ;
   un écran (16) connecté à l'ordinateur ;
   deux dispositifs de commande (20) connectés à l'ordinateur, chaque dispositif de commande étant doté d'une base (22), d'un bras (24), d'un outil final (30), où le bras combine plusieurs articulations (26), les deux dispositifs de commande étant fixés sur le châssis de telle façon que la première articulation de chaque bras est positionnée sous la base pendant l'utilisation, où chacun des dispositifs de commande fournit un signal de position à l'ordinateur, ce signal de position indiquant la position du bras du dispositif de commande ; où l'ordinateur est programmé pour :

      accepter un signal de position de chacun des dispositifs de commande ; et transforme chaque signal de position en position d'un outil chirurgical virtuel représenté sur l'écran grâce à une matrice de transformation de simulation déterminée par les opérations suivantes :
      déterminer une première matrice de transformation 4X4 entre un espace de travail de chaque dispositif de commande et un espace de travail du manipulateur ;
      déterminer une deuxième matrice de transformation 4X4 entre un espace de travail du manipulateur et un espace de travail du robot esclave ;
      déterminer la matrice de transformation de simulation entre l'espace de travail du dispositif de commande et la matrice du robot esclave en multipliant les première et deuxième matrices de transformation 4X4.

2. Le système de simulation du robot chirurgical de la revendication 1 où l'ordinateur est aussi programmé pour :
   utiliser une fonction de transformation mathématique pour modifier la relation entre le mouvement du bras dans l'espace réel et le mouvement de l'outil chirurgical virtuel dans un espace virtuel.

3. Le système de simulation du robot chirurgical de la revendication 2, où la fonction de transformation mathématique définit les relations du bras et des mouvements de l'outil chirurgical virtuel pour reproduire en très grande partie les relations de positionnement d'une commande en positions d'un outil chirurgical dans un robot chirurgical.

4. Le système de simulation du robot chirurgical de la revendication 3 où le robot chirurgical est un système chirurgical da Vinci®.

5. Le système de simulation du robot chirurgical de la revendication 1 dont l'écran est tactile.

6. Le système de simulation du robot chirurgical de la revendication 1 inclut aussi une commande au pied (40).

7. Le système de simulation du robot chirurgical de la revendication 1 dont le châssis est réglable.

8. Le système de simulation du robot chirurgical de la revendication 7 dont le châssis peuvent être plié pour devenir principalement plat.

9. Le système de simulation du robot chirurgical de la revendication 1 est équipé d'un écran binoculaire (32) capable d'afficher une image stéréoscopique.

10. Le système de simulation du robot chirurgical de la revendication 1 où chaque dispositif de commande autorise au minimum un déplacement libre sur six degrés.

11. Une méthode de simulation de la cinématique d'un système de chirurgie robotisée incluant les étapes suivantes :
    Fournir un système de simulation de robot (510) incluant :

       un ordinateur ;
       un écran connecté à l'ordinateur ;

un dispositif de commande connecté à l'ordinateur, le dispositif de commande doté d'un espace de travail défini par l'ampleur des mouvements du dispositif de commande ; fournir un (520) système de chirurgie robotisée incluant :

une console principale incluant un manipulateur, un espace de travail pour le manipulateur défini par l'enveloppe des mouvements du manipulateur ; et un robot esclave qui inclut un espace de travail pour le robot esclave défini par l'enveloppe des mouvements du robot esclave ;

déterminer une première (530) matrice de transformation 4X4 entre l'espace de travail du dispositif de commande et l'espace de travail du manipulateur ;

déterminer (540) une deuxième matrice de transformation 4X4 entre l'espace de travail du manipulateur et l'espace de travail du robot esclave ;

déterminer (550) une matrice de transformation de simulation entre l'espace de travail du dispositif de commande et la matrice du robot esclave en multipliant la première et la deuxième matrices de transformation 4X4 ; et

utiliser (560) la matrice de transformation de simulation pour que l'outil chirurgical virtuel représenté à l'écran réponde à un changement de position du dispositif de commande et simule en grande partie une réaction du robot esclave à un changement de position du manipulateur.

FIG. 1A

FIG. 1B

FIG. 2

FIG. 3A

FIG. 3B

FIG. 4A

FIG. 4B

FIG. 5A

FIG. 5B

FIG. 6A

FIG. 6B

FIG. 6C

FIG. 6D

FIG. 7A

FIG. 7B

FIG. 8A

FIG. 8B

FIG. 9

FIG. 10A

FIG. 10B

500

PROVIDING A ROBOT SIMULATION SYSTEM. — 510

PROVIDING A ROBOTIC SURGERY SYSTEM. — 520

DETERMINING A FIRST 4X4 TRANSFORMATION MATRIX BETWEEN THE INPUT DEVICE WORKSPACE AND THE MANIPULATOR WORKSPACE. — 530

DETERMINING A SECOND 4X4 TRANSFORMATION MATRIX BETWEEN THE MANIPULATOR WORKSPACE AND THE SLAVE ROBOT WORKSPACE. — 540

DETERMINING A SIMULATION TRANSFORMATION MATRIX BETWEEN THE INPUT DEVICE WORKSPACE AND THE SLAVE ROBOT WORKSPACE BY MULTIPLYING THE FIRST AND THE SECOND TRANSFORMATION MATRICES. — 550

USING THE SIMULATION TRANSFORMATION MATRIX TO CAUSE A VIRTUAL SURGICAL TOOL DEPICTED ON THE DISPLAY TO RESPOND TO A POSITIONAL CHANGE IN THE INPUT DEVICE WHICH SUBSTANTIALLY SIMULATES A RESPONSE OF THE SLAVE ROBOT TO A POSITIONAL CHANGE IN THE MANIPULATOR. — 560

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Advanced da Vinci surgical system simulator for surgeon training and operation planning. *International Journal of Medical Robotics and Computer Assisted Surgery,* 19 June 2007, vol. 3 (3), 245-251